# EUROPEAN PATENT APPLICATION

(11) **EP 4 088 652 A1**
(43) Date of publication of application: **16.11.2022**
(21) Application number: 21305613.8
(22) Date of filing: 11.05.2021
(51) Int. Cl.: A61B 5/00, A61B 5/316, G16H 20/10, G16H 40/67, G16H 10/20, G16H 15/00, G16H 40/63

(54) **MANAGEMENT OF INFORMATION FROM ACTIVE IMPLANTABLE MEDICAL DEVICE**

(71) Applicant: Implicity, 92120 Montrouge (FR)
(72) Inventor: GENTILS, Marika, 92120 Montrouge (FR); DANOUN, Kaissa, 92120 Montrouge (FR); KLAES, Stefan, 92120 Montrouge (FR); PERLMUTTER, David, 92120 Montrouge (FR); ROSIER, Arnaud, 92120 Montrouge (FR); DURAND, Julien, 92120 Montrouge (FR)
(74) Representative: Icosa

(57) **Abstract**

The present invention relates to a computer-implemented method for managing information relating to at least one patient with an active implantable medical device (AIMD) said method comprising, for each patient: receiving (110) at least one manufacturer information (20) representative of an output of the respective AIMD over time; analyzing (120) the received manufacturer information based on at least one first type rule (21), each first type rule (21) being associated to a predefined first sensor event and to at least one patient's clinical information (22) relating to a patient medical status, the analyzing comprising applying each first type rule (21) to the received manufacturer information (20) in order to determine, depending on each corresponding patient's clinical information (22) of the patient, whether the corresponding predefined first sensor event has occurred and is relevant for diagnosis; labeling (130) the manufacturer information (20) based on a result of the analyzing; outputting data representative of the labeled manufacturer information (30) for diagnosis taking account of the predefined first sensor event occurrence and relevancy.

## Description

### FIELD OF INVENTION

The present invention pertains to the field of analysis of data collected from an active implantable medical device. In particular, the invention relates to a method and a system for managing information relating to at least one patient with an active implantable medical device (AIMD) collecting data relating to the physical and/or physiological activity of said patient and in general signals recorded by the sensors from said AIMD.

### BACKGROUND OF INVENTION

Nowadays active implantable medical devices are commonly used to treat and/or monitor a variety of medical conditions. Such AIMDs may store and periodically transmit outside the body information relating to the operation of the device for analysis, programming, and/or the like. More particularly, AIMDs store and transmit information for in office or remote monitoring by a healthcare provider. Remote monitoring of patients having a AIMD advantageously provides patient follow-up and outcome by allowing early detection of abnormalities in a physiological activity of the patient and/or technical problems with the devices. However, each AIMD in each patient generates a large number of data relating to the physical and/or physiological activity of the patient and the state of the device itself which makes the follow-up and management of these data a timeconsuming task for healthcare providers. Furthermore, healthcare providers are often responsible for a large number of patients. In this context, there is a necessity of reducing the workload of healthcare providers.

It is with these observations in mind, among others, that various aspects of the present disclosure were conceived and developed.

### SUMMARY

The present invention relates to a system for managing information relating to at least one patient with an active implantable medical device (AIMD) comprising at least one sensor, said system comprising:
- at least one input adapted to receive at least one manufacturer information representative of an output of the respective AIMD over time;
- at least one processor configured to:
   ∘ analyze the received manufacturer information based on at least one first type rule, each first type rule being associated to a predefined first sensor event and to at least one patient's clinical information relating to a patient medical status, the analyzing comprising applying each first type rule to the received manufacturer information in order to determine, depending on each corresponding patient's clinical information of the patient, whether the corresponding predefined first sensor event has occurred and is relevant for diagnosis;
   ∘ provide at least one label associated to the manufacturer information based on a result of the analysis;
at least one output adapted to provide data representative of labeled manufacturer information for diagnosis taking account of the predefined first sensor event occurrence and relevancy.

Advantageously, the present invention allows to select among the sensor event that has occurred only the once that are relevant for diagnosis.

According to one embodiment, the system is comprised into a remote monitoring platform.

According to one embodiment, said at least one processor is further configured to record the labeled manufacturer information labelled as not relevant and to exploit the labeled manufacturer information labelled as relevant.

According to one embodiment:
- the manufacturer information comprises at least one alert information relating to a suspected abnormal activity of the patient's heart, each alert information being associated to a time stamp representing the time at which the alert information is generated;
- the patient's clinical information comprises at least one intake of a drug and a corresponding drug intake time interval during which the patient takes said drug;
said at least one processor is further configured, for each patient, to applying said first type rule by comparing each time stamp to the drug intake time interval, so that whenever the time stamp is comprised in said drug intake time interval, the manufacturer information corresponding to said time stamp is automatically labeled as labeled manufacturer information not to be notified by the remote monitoring platform. This embodiment, advantageously allows to reduce the number of alert that are generated by the device manufacturer platform, but removing on the base of the patient clinical information the alert that are not relevant for the diagnosis of the patient.

According to one embodiment, the alert information includes an alert relating to atrial fibrillation burden, and the drug is an anticoagulant drug.

According to one embodiment, the patient's clinical information is at least one of the following: age, sex, medical scores or classes, weight, comorbidities, symptoms, underwent surgeries, left ventricular fraction ejection.

According to one embodiment, for each patient, the manufacturer information further comprises at least one sensor information representative of a physiological and/or physical activity of the patient as a function of time, the analysis of the received manufacturer information further comprising applying at least one second type rule to said sensor information in order to determine whether a predefined second sensor event, associated to said second type rule, has occurred.

According to one embodiment, said at least one processor is further configured to notify at least one label associated to the manufacturer information labeled on the basis of each second type rule, said at least one label being representative of each corresponding predefined second sensor event detected.

According to one embodiment, for each patient, the manufacturer information comprises:
- at least one alert information relating to a suspected abnormal activity of the patient's heart, each alert information being associated to a time stamp representing the time at which the alert information is generated, and
- for each alert information, at least one sensor information used to generate the alert information;
- the patient's clinical information comprises at least one intake of a drug and a corresponding drug intake time interval during which the patient takes said drug;
and wherein the at least one processor is further configured to apply at least one third type rule to the received manufacturer information, each third type rule being associated to a predefined third sensor event, and to at least one predefined threshold value, the applying each third type rule to the received manufacturer information including:
- comparing each time stamp to the drug intake time interval; and
- comparing a predetermined portion of each sensor information to the predefined threshold value,
so that, whenever the time stamp belongs to said drug intake time interval and the predetermined portion of the sensor information is lower or higher than the predefined threshold value, the manufacturer information corresponding to said time stamp is automatically labeled as manufacturer information not to be notified.

The present invention also relates to a computer-implemented method for managing information relating to at least one patient with an active implantable medical device, said method comprising, for each patient:
- receiving at least one manufacturer information representative of an output of the respective AIMD over time;
- analyzing the received manufacturer information based on at least one first type rule, each first type rule being associated to a predefined first sensor event and to at least one patient's clinical information relating to a patient medical status, the analyzing comprising applying each first type rule to the received manufacturer information in order to determine, depending on each corresponding patient's clinical information of the patient, whether the corresponding predefined first sensor event has occurred and is relevant for diagnosis;

- labeling the manufacturer information based on a result of the analyzing;
outputting data representative of the labeled manufacturer information for diagnosis taking account of the predefined first sensor event occurrence and relevancy.

According to one embodiment, for each patient:
- the manufacturer information comprises at least one alert information relating to a suspected abnormal activity of the patient's heart, each alert information being associated to a time stamp representing the time at which the alert information is generated;
- the patient's clinical information comprises at least one intake of a drug and a corresponding drug intake time interval during which the patient takes said drug;
for each first type rule, the analysis comprises applying said first type rule by comparing each time stamp to the drug intake time interval, so that whenever the time stamp is comprised in said drug intake time interval, the manufacturer information corresponding to said time stamp is automatically labeled as labeled manufacturer information not to be notified.

According to one embodiment, the alert information includes an alert relating to an atrial fibrillation burden, and the drug is an anticoagulant drug.

According to one embodiment, for each patient, the manufacturer information further comprises at least one sensor information representative of a physiological and/or physical activity of the patient as a function of time, the analysis of the received manufacturer information further comprising applying at least one second type rule to said sensor information in order to determine whether a predefined second sensor event, associated to said second type rule, has occurred.

According to one embodiment, the predefined second sensor event comprises at least one of the following medical conditions: first episode of atrial fibrillation, increasing paroxysmal atrial fibrillation, paroxysmal atrial fibrillation, persistent atrial fibrillation, and end of persistent atrial fibrillation.

According to one embodiment, the at least one sensor information includes an atrial fibrillation burden as a function of time and/or a biventricular pacing percentage as a function of time, derived from the active implantable medical device.

According to one embodiment, for each patient, the manufacturer information comprises:
- at least one alert information relating to a suspected abnormal activity of the patient's heart, each alert information being associated to a time stamp representing the time at which the alert information is generated, and
- for each alert information, at least one sensor information used to generate the alert information;
- the patient's clinical information comprises at least one intake of a drug and a corresponding drug intake time interval during which the patient takes said drug;
and the analysis of the received manufacturer information further comprises applying at least one third type rule to the received manufacturer information, each third type rule being associated to a predefined third sensor event, and to at least one predefined threshold value, the applying each third type rule to the received manufacturer information including:
- comparing each time stamp to the drug intake time interval; and
- comparing a predetermined portion of each sensor information to the predefined threshold value,
so that, whenever the time stamp belongs to said drug intake time interval and the predetermined portion of the sensor information is lower or higher than the predefined threshold value, the manufacturer information corresponding to said time stamp is automatically labeled as manufacturer information not to be notified.

The present invention also relates to a computer program product for managing information relating to at least one patient with an active implantable medical device, the computer program product comprising instructions which, when the program is executed by a computer, cause the computer to carry out the steps of the method according to any one of the embodiments described here above.

The present invention also relates to a non-transitory computer-readable storage medium comprising instructions which, when executed by a computer, cause the computer to carry out the steps of the method according to any one of the embodiments disclosed hereabove.

### DEFINITIONS

In the present invention, the following terms have the following meanings:
- **"Atrial Fibrillation burden"** is the proportion of time an individual is in Atrial Fibrillation during a monitoring period, notably one day, expressed as a percentage.
- **"Processor"** should not be construed to be restricted to hardware capable of executing software, and refers in a general way to a processing device, which can for example include a computer, a microprocessor, an integrated circuit, or a programmable logic device (PLD). The processor may also encompass one or more Graphics Processing Units (GPU), whether exploited for computer graphics and image processing or other functions. Additionally, the instructions and/or data enabling to perform associated and/or resulting functionalities may be stored on any processor-readable medium such as, e.g., an integrated circuit, a hard disk, a CD (Compact Disc), an optical disc such as a DVD (Digital Versatile Disc), a RAM (Random-Access Memory) or a ROM (Read-Only Memory). Instructions may be notably stored in hardware, software, firmware or in any combination thereof.
- **"Active Implantable Medical Device (AIMD)"** refers any active medical device which is intended to be totally or partially introduced, surgically or medically, into the human body or by medical intervention into a natural orifice, and which is intended to remain after the procedure, and encompassing a source of energy. In the context of the present invention the AIMD are preferably devices intended to monitor and/or regulate the cardiac rhythm of the patient. Said devices include for instance pacemakers, implantable cardioverter defibrillators (ICDs), cardiac resynchronization therapy (CRT) devices, cardiac pump (e.g. left ventricular assist device (LVAD), artificial heart), insertable cardiac monitors (ICM), neurostimulators and the like. In general, AIMD may also store and transfer their data to a device manufacturer platform for further analysis. The AIMD in the context of the present invention may notably comprise at least one sensor related to at least one physical or physiological parameter from the following list: cardiac rhythmic status, ventilation, temperature, ventricular or arterial pressure, myocardial activity, neural activity, cardiac hemodynamics, cardiac mechanics and the like. The sensor is at least one of the following: electrocardiogram (ECG), electrogram (EGM), accelerometer, transthoracic or intrathoracic impedance, thermistor, MEMs, electromyogram (EMG), electroneurogram (ENG), and the like.
- **"Device manufacturer platform"** refers to software and servers provided by the manufacturer of the AIMD. Said device manufacturer platform is configured to store the data received from the AIMDs implanted into multiple patients and perform further analysis of the data before displaying the results of this analysis to healthcare professionals or transferring the results of this analysis to an external receiver. Data from AIMD may also be available for third party software intended to provide healthcare professionals with additional services.
- **"Remote monitoring platform"** refers to any system for data management configured to receive and store and/or analyze data received from at least one device manufacturer platform corresponding to at least one patient equipped with an AIMD. The term "remote" refers to the fact that the monitoring platform is remote with respect to the subject. In one example, said remote monitoring platform may, in general, receive data from one or more AIMD of patients to manage the care of those patients, including receipt of data, reports, and information from such devices to enable a healthcare professional to view, document, report on the health status of the patients. Such data may be received at the remote monitoring platform through many sources, including the corresponding device, a device programming machine, reporting from the patient or a manufacturer, or from a third-party entity receiving the data from the device. In this example, the remote monitoring platform may also provide one or more interfaces through which healthcare professionals or other users of the platform may manage the receipt of the AIMD data.

- **"Manufacturer alert"** is a manufacturer information generated by the device manufacturer platform from the data outputted by the AIMD. Said manufacturer alert is generated using a detection method on the data outputted by the AIMD and is intended to be notified so as to raise awareness of healthcare professionals about suspected abnormal activity from at least one AIMD sensor, related to a specific physical or physiological parameter of the said patient. The manufacturer alert comprises at least: a timestamping corresponding to the time at which the suspected abnormal activity detected from at least one AIMD sensor, related to a specific physical or physiological parameter has taken place and a description of the suspected detected abnormal activity.
- **"Notification"** refers to a message information sent to healthcare professionals by email, SMS or third-party entity, intended to increase the likelihood of the healthcare professionals receiving the message in a short delay.
- **"Manufacturer information"** refers to information relative to an AIMD from a certain manufacturer and being forwarded by said device manufacturer platform to physicians or a third-party entity. Said manufacturer information may comprise measurements (e.g. statistics, temporal curves, histogram distributions), alerts (e.g. types, timestamps), episodes (e.g. time-series data, timestamps, markers).
- **"Manufacturer episode"** refers to a portion of a sensor signal having finite time duration which has been identified and recorded by the AIMD. Indeed, the device manufacturer may implement in the said AIMD an identification method to perform a preliminary analysis on the sensed signal so as to identify a portion of sensor signal associated to a physical or physiological (e.g., cardiac) abnormal activity and then record it. Said portion of sensor signal may be as well recorded if a patient triggers the recording. The episode comprises, in addition to a portion of sensor signal, also a timestamp of the recording (day and time) and the type of recording (i.e., patient or identification method or triggered episode).
- **"Label"** refers to the recontextualization of the manufacturer information.
- **"Rule"** refers to a determinate method for performing a mathematical operation and obtaining a certain result, in the case of the present invention using the manufacturer information and/or patient's clinical information.

### LIST OF FIGURES

The present disclosure will be better understood, and other specific features and advantages will emerge upon reading the following description of particular and non-restrictive illustrative embodiments, the description making reference to the annexed drawings wherein:
**Figure 1** is a block diagram representing schematically a particular mode of a system for managing information relating to at least one patient with an active implantable medical device, compliant with the present disclosure.
**Figure 2** is a block diagram representing schematically a particular mode of use of the system for managing information according to a second embodiment further comprising a second type rule.
**Figure 3** is a representation of the cardiac information AF burden as a function of time annotated with the labels that are associated by the system to the corresponding manufacturer information according to the second embodiment.
**Figure** 4 is a block diagram representing schematically a particular mode of use of the system for managing information according to a third embodiment further comprising a third type rule.
**Figure 5** is a block diagram representing schematically a particular mode of use of the system for managing information.

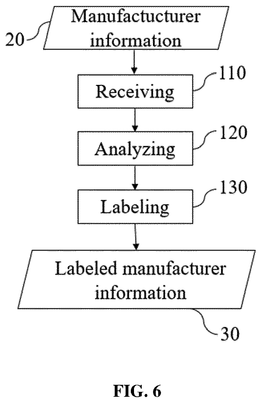

**Figure 6** is a flow chart showing successive steps executed with the system for managing of figure 1.

On the figures, the drawings are not to scale, and identical or similar elements are designated by the same references.

### ILLUSTRATIVE EMBODIMENTS OF THE INVENTION

The present description illustrates the principles of the present disclosure. It will thus be appreciated that those skilled in the art will be able to devise various arrangements that, although not explicitly described or shown herein, embody the principles of the disclosure and are included within its spirit and scope.

All examples and conditional language recited herein are intended for educational purposes to aid the reader in understanding the principles of the disclosure and the concepts contributed by the inventor to furthering the art, and are to be construed as being without limitation to such specifically recited examples and conditions.

Moreover, all statements herein reciting principles, aspects, and embodiments of the disclosure, as well as specific examples thereof, are intended to encompass both structural and functional equivalents thereof. Additionally, it is intended that such equivalents include both currently known equivalents as well as equivalents developed in the future, i.e., any elements developed that perform the same function, regardless of structure.

Thus, for example, it will be appreciated by those skilled in the art that the block diagrams presented herein may represent conceptual views of illustrative circuitry embodying the principles of the disclosure. Similarly, it will be appreciated that any flow charts, flow diagrams, and the like represent various processes which may be substantially represented in computer readable media and so executed by a computer or processor, whether or not such computer or processor is explicitly shown.

The functions of the various elements shown in the figures may be provided through the use of dedicated hardware as well as hardware capable of executing software in association with appropriate software. When provided by a processor, the functions may be provided by a single dedicated processor, a single shared processor, or a plurality of individual processors, some of which may be shared.

It should be understood that the elements shown in the figures may be implemented in various forms of hardware, software or combinations thereof. Preferably, these elements are implemented in a combination of hardware and software on one or more appropriately programmed general-purpose devices, which may include a processor, memory and input/output interfaces.

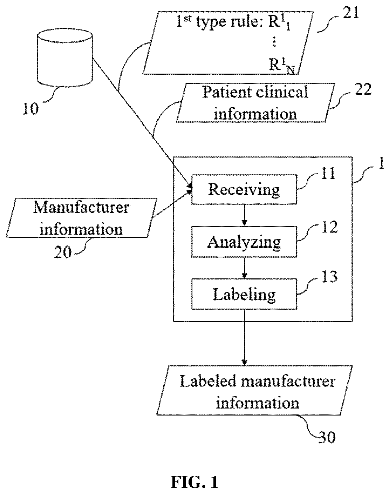

The present disclosure will be described in reference to a particular functional embodiment of a system 1 for managing information relating to at least one patient with an active implantable medical device, as illustrated on Figure 1.

The system 1 is adapted to produce labeled manufacturer information 30 from manufacturer information 20 representative of an output of the respective AIMD over time and from at least one patient's clinical information 22 relating to a patient's medical status. The labeled manufacturer information 30 may comprise the manufacturer information 20 which have been analyzed and the label generated based on the result of the analysis.

The manufacturer information 20 may be directly received from the AIMD which transmits the manufacturer information 20 via a communication network. Alternatively, the manufacturer information 20 may be received by the device manufacturer's platform that has previously received the output of the AIMD over time and eventually, performed an analysis of said output.

The manufacturer information 20 may comprise different sort of data. Notably the manufacturer information may comprise measurements recorded by the one or more sensors comprised in the AIMD and/or parameters calculated by the device manufacturer's platform using said recorded measurement. The recorded measurements or the calculated parameters, related to the condition of the at least one parameter measured by the at least one sensor of the AIMD, are also called in the present description sensor information. The recorded measurements or the calculated parameters, related to the condition of the AIMD (i.e., battery level and the like), are also called in the present description device information. The manufacturer information 20 may further comprise alerts that have been generated by the device manufacturer's platform using said recorded measurement and/or calculated parameters.

Though the presently described system 1 is versatile and provided with several functions that can be carried out alternatively or in any cumulative way, other implementations within the scope of the present disclosure include system having only parts of the present functionalities.

The system 1 is advantageously an apparatus, or a physical part of an apparatus, designed, configured and/or adapted for performing the mentioned functions and producing the mentioned effects or results. In alternative implementations, the system 1 is embodied as a set of apparatus or physical parts of apparatus, whether grouped in a same machine or in different, possibly remote, machines. The system 1 may, for example, have functions distributed over a cloud infrastructure and be available to users as a cloud-based service, or have remote functions accessible through an API.

In what follows, the modules are to be understood as functional entities rather than material, physically distinct, components. They can consequently be embodied either as grouped together in a same tangible and concrete component, or distributed into several such components. Also, each of those modules is possibly itself shared between at least two physical components. In addition, the modules are implemented in hardware, software, firmware, or any mixed form thereof as well. They are preferably embodied within at least one processor of the system 1.

The system 1 comprises a receiving module 11 for receiving the manufacturer information 20 and the patient's clinical information 22, as well as the first type rule, second type rule and/or the third type rule 21 stored in one or more local or remote database(s) 10. The latter can take the form of storage resources available from any kind of appropriate storage means, which can be notably a RAM or an EEPROM (Electrically-Erasable Programmable Read-Only Memory) such as a Flash memory, possibly within an SSD (Solid-State Disk). In advantageous embodiments, the first type rule, second type rule and/or the third type rule 21 have been previously defined. Alternatively, the first type rule, second type rule and/or the third type rule 21 are received from a communication network.

The first type rule, second type rule and/or the third type rule 21 may be defined by a logic rule, a predefined or variable threshold, a finite-state machine or a machine learning model.

The system 1 further comprises optionally a preprocessing module (not illustrated) for preprocessing the received manufacturer information 20 and possibly the patient's clinical information 22. Said preprocessing module may be configured to verify that the received manufacturer information 20 has the desired predefined format and that is consistent. If it is not the case, the preprocessing module may further convert the manufacturer information 20 into the desired predefined format. This embodiment advantageously allows to analyze manufacturer information 20 obtained from different manufacturers, which may use different format for the information they provide.

The system 1 also comprises an analysis module 12 for analyzing the received manufacturer information 20 based on at least one first type rule 21. Each of the first type rule 21 is associated to one predefined first sensor event and to at least one patient's clinical information 22 relating to a patient medical status. The analysis module 12 is configured to apply each first type rule 21 to the received manufacturer information 20 in order to determine, depending on each corresponding patient's clinical information 22 of the patient, whether the corresponding predefined first sensor event has occurred and is relevant for the diagnosis.

The system 1 further comprises a labelling module 13 for labeling the manufacturer information 20 based on a result of the analysis module 12 and for outputting these labeled manufacturer information 30 to a remote monitoring platform 43.

It may be observed that the operations by the modules 11, 12 and 13 are not necessarily successive in time, and may overlap, proceed in parallel or alternate, in any appropriate manner. For example, new manufacturer information 20 may be progressively received over time and analyzed, while the labelling module 13 is dealing with the previously obtained analysis results. In alternative examples, a batch of manufacturer information 20 corresponding to a multiple days sequence of manufacturer information 20 may be fully received and analyzed before it is submitted to the labelling module 13.

The system 1 is interacting with a remote monitoring platform 43, via which information can be entered and retrieved by a user. The remote monitoring platform 43 includes any means appropriate for entering or retrieving data, information or instructions, notably visual, tactile and/or audio capacities that can encompass any or several of the following means as well known by a person skilled in the art: a screen, a keyboard, a trackball, a touchpad, a touchscreen, a loudspeaker, a voice recognition system.

The manufacturer information 20 may comprise at least one alert information relating to a suspected abnormal activity of the patient's heart. Said alert information is the result of an analysis of the output of the active implantable medical device. This analysis has been performed in the AIMD itself or by the device manufacturer's platform. Hence the alert information comprises at least one alert and a time stamp representing the time at which the alert information has been generated.

The patient's clinical information 22 relative to a medical status of the patient may comprise information collected independently from the manufacturer information 20, such as information recording the medical prescriptions of the patient comprising at least one intake of a drug and a corresponding drug intake time interval during which the patient takes said drug, or other patient information such as, but not limited to, his/her sex, age, weight, comorbidities (e.g. hypertension, diabetes), measurements (e.g. left ventricular ejection fraction), a medical score (e.g. NYHA class, CHA₂DS₂-VASc score etc.), symptoms (e.g. pain), undergone surgeries, data obtained from a connected device measuring a physiological parameter and/or signal of the patent, data from electronic health record and the like.

In one embodiment, the first type rule 21 is defined in order to verify the medical relevance of the alert information received while taking into consideration the patient's clinical information 22. The first type rule may be defined by at least one logic rule, a predefined threshold, a finite-state machine or a machine learning model. Notably a method of clustering (i.e., decision tree, ontology, machine learning) may be used on the patient clinical information 22.

Notably, when executed by the analysis module 12, the first type rule 21 compares each time stamp of the alert information to the drug intake time interval of the patient's clinical information 22 in order to verify which time stamp belongs to the drug intake time interval. Then, the processor automatically labels the manufacturer information 20 that satisfies this first type rule 21 as labeled manufacturer information 30 "not to be notified" by the remote monitoring platform 43. This manufacturer information 30 carrying the label "not to be notified" is outputted so as to be received by the remote monitoring platform 43. All the manufacturer information 30 carrying the label "not to be notified" may be interpreted by the remote monitoring platform 43 as not relevant for the medical professionals and therefore will not trigger a notification to the healthcare professional. More in details, this manufacturer information 30 carrying the label "not to be notified" may be still accessible to the medical professionals via the remote monitoring platform 43 (i.e. the manufacturer information 30 carrying the label "not to be notified" may be recorded in the remote monitoring platform 43), but they will not produce a notification. Advantageously, this embodiment allows to reduce the quantity of alerts displayed to the healthcare professional while preserving the quality of care.

In one advantageous example, the alert information includes one alert relating to atrial fibrillation (AF) burden, notably a day-level AF burden, and the patient clinical information is the anticoagulation status of the patient (whether or not he is on anticoagulation therapy by taking an anticoagulant drug). The received manufacturer alerts may be AF burden alerts triggered when the day-level AF burden exceeds a predefined threshold. However, when the patient is taking anticoagulant drugs, the thromboembolic risk is significantly reduced and therefore the alert generated by a day-level AF burden which is greater than the predefined threshold is no longer relevant to evaluate the thromboembolic risk for the patient. This advantageously allow the medical professionals not to be notified with repeated AF burden manufacturer alerts for a patient already treated with anticoagulant drug, therefore significantly reducing the global amount of notification to be scrutinized by the medical professionals.

In one example, on the one hand, the patient's clinical information 22 comprises at least one symptom associated to a time stamp representing the time at which the patient has experienced said symptom. The information concerning the symptom and the time at which it had occurred may be provided directly by the patient via a patient interface (i.e. by pushing a trigger button, a smart phone application) which is configured to transfer the information to the system 1. On the other hand, if any, a manufacturer information related to a first sensor event with a time stamp is configured to transfer the information to the system 1. In this example, the first type rule is configured to compare the time stamp associated to the symptom with the time stamp associated to the manufacturer alert, based of at least one first sensor event. In this case, if the interval between these two time stamps is less than a predefined interval, then the two alerts are associated and labelled as "linked". For example, this patient's event can be related with a concomitant sensor event to analyze the origin of the faint (e.g. cardiac arrhythmia, vagal discharge). This event can be related with concomitant events from at least one sensor to analyze the origin of the faint (e.g. cardiac arrhythmia, vagal discharge).

In another example, the AIMD is a pacemaker configured to regularly measure and record intrathoracic impedance between the end of the lead and the corresponding pacemaker case. Said intrathoracic impedance is used in the device manufacturer's platform to generate impedance alert information concerning the risk of acute lung edema due to heart failure decompression (i.e., first cardia event). In this example, the patient's clinical information 22 comprises at least one evaluation of the heart failure degree associated to a time interval or at least one syndrome of the patient susceptible to cause heart failure, decompensation or worsening which provides a knowledge on the probability of acute lung edema in one time interval. The patient's clinical information 22 may as well comprise a value of the left ventricular ejection fraction measured for a time interval (i.e., previously measured by echocardiogram). The associated first type rule is configured to comparing each time stamp of the manufacturer heart failure-related sensor (e.g. transthoracic impedance or left ventricular mechanical activity measured with an accelerometer) alert to the time interval of the probability of acute lung edema and/or the time interval for the value of the measured left ventricular ejection fraction. If the probability of acute lung edema is low and/or the value of the left ventricular ejection fraction is normal, the execution of the first type rule causes the processor to automatically labels as "not to be notified" each manufacturer information corresponding to one time stamp comprised in said time interval(s).

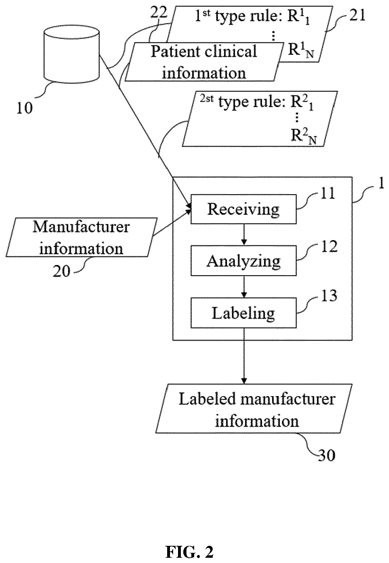

Figure 2 illustrates a second embodiment wherein the manufacturer information 20 further comprises at least one sensor information representative of a physiological or physical activity of the patient's as a function of time and the receiving module 11 further receive at least one second type rule. When executed by the processor, the second type rule is applied to the sensor information in order to determine whether a predefined second sensor event, associated with said one second type rule, has occurred. Notably, this second sensor event is an abnormal activity of the patient's heart rhythm. The result of the analysis according to this second type rule is then used by the labelling module 13 to label the manufacturer information 20 with a label providing medical relevant information on the detected second sensor event. Therefore, these labels, inversely to the "not to be notified label" of the first type rule, are intended to be notified to the medical professional via the remote monitoring platform 43. This embodiment allows advantageously to detect the occurrence of relevant sensor events which are not detected by the device manufacturer platform.

In one embodiment, the at least one sensor information is a cardiac information representative of an activity of the patient's heart as a function of time. The at least one cardiac information may be the atrial fibrillation burden as a function of time and/or the biventricular pacing percentage as a function of time, derived from the active implantable medical device.

In one embodiment, the second type rule comprises a predefined threshold associated to one sensor information and a control duration during which the sensor information is compared to the predefined threshold. The threshold may be not predefined but being a function of time according to a model. Alternatively, the second type rule may be a classifier such as a finite-state machine.

Multiple second type rules may be defined in order to detect the occurrence of the following predefined second sensor events using the AF burden as cardiac information: first occurrence of atrial fibrillation, increasing paroxysmal atrial fibrillation, paroxysmal atrial fibrillation, persistent atrial fibrillation, and end of persistent atrial fibrillation. Preferably, in this case, the AF burden is used as cardiac information.

In one example, one predefined second type of rule may comprise two predefined thresholds for the AF burden value provided. The first threshold may have a value comprised between 1% and 50% and the second threshold may have a value comprised between 50% and 100%. In one preferred example, the first threshold may have a value comprised between 1% and 45% and the second threshold between 55% and 100%, so that three ranges of AF burden values are defined for any combination of values of the first and second threshold.

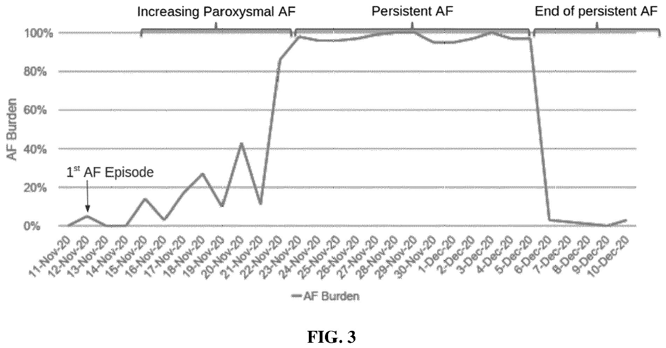

The second type rule may be defined to detect a persistent atrial fibrillation so that whenever the AF burden value is higher than the second threshold for a control duration comprised between 5 and 10 days, the AF burden values exceeding said second predefined threshold are labeled as "persistent atrial fibrillation" (Fig. 3).

This second type rule may be further configured to detect the "paroxysmal AF" by verify that the AF burden value is comprised between the first threshold and the second threshold for a control duration superior of 7 days.

The "end of persistent atrial fibrillation" may be detected by this second type rule when is configured to verify that the AF burden value has decreased below the second threshold after that a previous manufacturer information 30 has been labeled as "persistent atrial fibrillation" (Fig. 3).

Furthermore, the occurrence of the first occurrence of atrial fibrillation is detected by a second type rule configured to detect the first atrial fibrillation burden value superior to zero for one patient. Therefore, when this second type rule is applied to the curve of AF burden as a function of time for one patient, the corresponding manufacturer information (i.e., the atrial fibrillation burden value) will be labelled as "first atrial fibrillation burden value", as shown in Figure 3.

A second type rule may be defined so as to label the manufacturer information as "increasing paroxysmal AF" whenever a rise of more than 10% to 50% of the average AF burden during a paroxysmal period (Fig. 3) is detected.

A second type rule may be defined so as to label the manufacturer information as "end of persistent atrial fibrillation", whenever AF burden is inferior than 5% for more than 7 days after an "persistent atrial fibrillation" has been detected (Fig. 3).

One second type rule may be configured to be applied on the biventricular pacing percentage as a function of time in order to detect a worsening of the ventricular synchronization. For example, a rise of the 30% of the biventricular pacing percentage during a 5 to 10 days may cause the related manufacturer information to be labeled as "improvement of the ventricular synchronization".

In one example, the at least one cardiac information is the intrathoracic impedance as a function of time. One second type rule may be defined so as to verify that the intrathoracic impedance does not switch to a higher value (intermittently). When the intrathoracic impedance switches to this higher value, this second type rule allows to detect the occurrence of a sensor event being the misfunctioning of the AIMD to be notified to the medical professional.

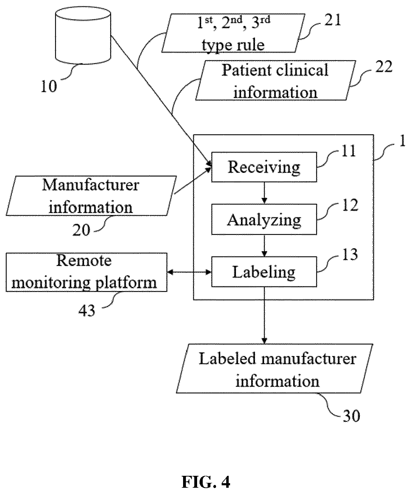

According to one embodiment, the manufacturer information 20 further comprises at least one alert information relating to a suspected abnormal activity of the patient's heart and, for each alert information, at least one cardiac information used to generate the alert information. In this embodiment, the patient's clinical information 22 comprises at least one intake of a drug and a corresponding drug intake time interval during which the patient takes said drug. The processor is configured to apply at least one third type rule 21 to the received manufacturer information 20, as shown in figure 4. Each of said third type rule 21 is associated to a predefined third sensor event and a specific drug taken by the patient which is supposed to have an influence on the cardiac information and alert information under analysis. More in detail, the third type rule 21 is such that when executed by the processor, the processor compares each time stamp of the alert information to the drug intake time interval of the patient's clinical information 22 and compares as well a predetermined portion of each cardiac information to the predefined threshold value. Then the processor automatically labels the received manufacturer information as "not to be notified" by the remote monitoring platform 43 whenever the time stamp belongs to said drug intake time interval and the predetermined portion of the cardiac information is lower than the predefined threshold value.

The implementation of this embodiment using one third type rule 21 is particularly advantageous in the context of the modification of a treatment for arrhythmia which may have as consequence a rise in the threshold value associated to the ventricular stimulation (i.e. third sensor event). Indeed, generally the alert information provided by the manufacturer are based on a specific fixed threshold.

According to one embodiment, the analysis module 12 is further configured to receive a fourth type rule allowing to establish a causality link between at least two information. In one embodiment, for each patient, the manufacturer information 20 further comprises at least one sensor information being at least one sensor episode associated to the time stamp representing the time of the episode recording and at least one alert information associated to the time stamp representing the time at which the alert information is generated. Said fourth type rule is then configured to associate the recorded manufacturer sensor episodes to the manufacturer alerts comprised in the alert information. Indeed, the episode detected by the AIMD triggers an alert to notify the physician, but in general the manufacturer alert does not contain proper information to identify directly the corresponding sensor episode (the alert and episode are transmitted as independent information). The analysis module 12 is configured to analyze the received manufacturer information by applying at least one fourth type rule 21 which compares the time stamps of each sensor episode to the time stamp of each manufacturer alert, so that whenever the interval between one episode time stamp and one alert time stamp is inferior to a predefined value (i.e., predefined value defined so as to associate synchronous episode and alert), the alert information and the episode are labelled with one identical label. The fourth rule advantageously comprises applying this comparison between the time stamps of a sensor episode and the time stamp of a manufacturer alert only among sensor episodes and manufacturer alerts which have been associated to a same class by the manufacturer platform. This example advantageously allows to establish a relationship between the manufacturer alerts and the manufacturer sensor episodes which triggered the generation of the related manufacturer alerts.

In one example for this fourth rule, the alert information comprises at least one manufacturer alert generated by the manufacturer platform and at least one patient triggered alert which is the alert generated by the patient when he/she indicates that is experiencing a symptom (i.e., an abnormal heart activity) for example by pushing an alert button or using a smartphone app. When a patient indicates that he/she experiencing a symptom a recording of an episode may be triggered some time before or after the generation of the patient triggered alert, however the device manufacturer's platform generally does not associate said episode to the corresponding patient triggered alert. One fourth type rule may be therefore defined to compare the time stamps of each manufacturer alert to the time stamps of the patient triggered alerts and the patient triggered episodes so that when the time stamps of one manufacturer alert is substantially synchronous to one patient triggered episode and one patient triggered episode, said manufacturer alert, said patient triggered alerts and said patient triggered episodes are labelled with one identical label. This example advantageously allows to establish a relation between the manufacturer alerts and patient triggered episodes, and the patient triggered alerts.

In one alternative embodiment, the fourth type rule is configured to establish a causality link in a sequence of successive sensor events and label the corresponding manufacturer alerts as "may be caused by" the relative past events. The information of the probability that one type of sensor event may be caused by a second type of sensor event may be used.

A fifth type rule may be defined for detecting alerts occurring with a regular frequency (e.g., every day at the same time) and which have been previously considered as nonrelevant for diagnosis of the patient independently from sensors information and/or patient clinical information, and labelling them as "not relevant alert".

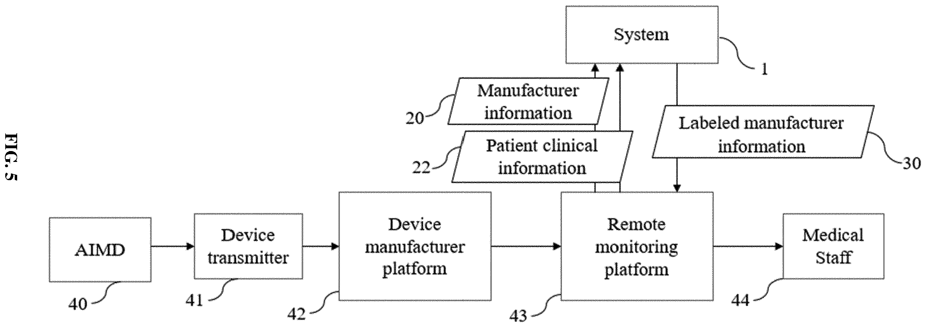

Figure 5 shows a particular mode of use of the system for managing information. As disclosed above, the AIMD 40 records one or more measurements which are transferred to a device transmitter 41, said transmission may be done through a wire or wirelessly. The measurements are then transferred via communication network to the device manufacturer's platform 42 which analyses the measurements producing the manufacturer information 20. Said manufacturer information are then sent to the remote monitoring platform 43 which may host as well the patient's clinical information 22. The remote monitoring platform 43 is dynamically connected to the system 1 so as to provide to the system 1 the manufacturer information 20 and the patient's clinical information 22, and receive the labeled manufacturer information 30. Then the remote monitoring platform 43, thanks to its means, provides the pertinent labeled manufacturer information 30 to the medical professional 44.

In operation, a process as described below may for example be executed, in relation with Figure 4. In a situation of a patient experiencing suspected abnormal heart activity, the heart activity of the patient is constantly monitored thanks to an AIMD which provides manufacturer information 20 representative of an output of the AIMD over time. This manufacturer information 20 are submitted to the system 1 for management. In addition, the patient's clinical information 22 relating to a patient medical status are provided to the system 1. From the manufacturer information 20 and the patient's clinical information 22, the system 1 automatically outputs the labeled manufacturer information 30 for the respective patient. This labeled manufacturer information is relied upon by medical professional, at least as aid, to follow-up the heart activity of the patient so as to perform appropriate diagnosis.

In its automatic actions, the system 1 may for example execute the following process (Figure 2):
- receiving at least one manufacturer information 20 representative of an output of the respective AIMD over time (step 110);
- analyzing the received manufacturer information based on at least one first type rule 21, each first type rule 21 being associated to a predefined first sensor event and to at least one patient's clinical information 22 relating to a patient medical status, the analysis comprising applying each first type rule 21 to the received manufacturer information 20 in order to determine, depending on each corresponding patient's clinical information 22 of the patient, whether the corresponding predefined first sensor event has occurred and is relevant for the diagnosis (step 120);
- labeling the manufacturer information 20 based on a result of the analysis (step 130);
- outputting data representative of labeled manufacturer information 30.

The data representative of the labeled manufacturer information 30 may be used by the remote monitoring platform 43.

## Claims

1. A system (1) for managing information relating to at least one patient with an active implantable medical device (AIMD) comprising at least one sensor, said system comprising:
- at least one input adapted to receive at least one manufacturer information (20) representative of an output of the respective AIMD over time;
- at least one processor configured to:
∘ analyze the received at least one manufacturer information (20) based on at least one first type rule (21), each first type rule (21) being associated to a predefined first sensor event and to at least one patient's clinical information (22) relating to a patient medical status, said analysis comprising applying each first type rule (21) to the received at least one manufacturer information (20) in order to determine, depending on each corresponding patient's clinical information (22) of the patient, whether the corresponding predefined first sensor event has occurred and is relevant for diagnosis;
∘ provide at least one label associated to the at least one manufacturer information (20) based on a result of said analysis;
- at least one output adapted to provide data representative of at least one labeled manufacturer information (30) for diagnosis taking account of the predefined first sensor event occurrence and relevancy.

2. The system according to claim 1, wherein the system is comprised into a remote monitoring platform (43).

3. The system according to either one of claim 1 or 2, wherein said at least one processor is further configured to record the at least one labeled manufacturer information (30) labelled as not relevant and to exploit the at least one labeled manufacturer information (30) labelled as relevant.

4. The system of claim according to any one of claims 2 to 3, wherein:
- the at least one manufacturer information (20) comprises at least one alert information relating to a suspected abnormal activity of the patient's heart, each alert information being associated to a time stamp representing the time at which the alert information is generated;
- the patient's clinical information (22) comprises at least one intake of a drug and a corresponding drug intake time interval during which the patient takes said drug;
- said at least one processor is further configured, for each patient, to applying said first type rule (21) by comparing each time stamp to the drug intake time interval, so that whenever the time stamp is comprised in said drug intake time interval, the manufacturer information (20) corresponding to said time stamp is automatically labeled as labeled manufacturer information (30) not to be notified by the remote monitoring platform (43).

5. The system according of claim 4, wherein the alert information includes an alert relating to atrial fibrillation burden, and the drug is an anticoagulant drug.

6. The system according of claim 1, wherein the patient's clinical information (22) is at least one of the following: age, sex, medical scores or classes, weight, comorbidities, symptoms, underwent surgeries, left ventricular fraction ejection.

7. The system according to any one of claims 1 to 6, wherein, for each patient, the manufacturer information (20) further comprises at least one sensor information representative of a physiological and/or physical activity of the patient as a function of time, the analysis of the received manufacturer information (20) further comprising applying at least one second type rule (21) to said sensor information in order to determine whether a predefined second sensor event, associated to said second type rule (21), has occurred.

8. The system according to claim 7, wherein said at least one processor is further configured to notify, via the remote monitoring platform, at least one label associated to the manufacturer information (30) labeled on the basis of each second type rule (21), said at least one label being representative of each corresponding predefined second sensor event detected.

9. A computer-implemented method for managing information relating to at least one patient with an active implantable medical device (AIMD), said method comprising, for each patient:
- receiving (110) at least one manufacturer information (20) representative of an output of the respective AIMD over time;
- analyzing (120) the received manufacturer information (20) based on at least one first type rule (21), each first type rule (21) being associated to a predefined first sensor event and to at least one patient's clinical information (22) relating to a patient medical status, the analyzing comprising applying each first type rule (21) to the received manufacturer information (20) in order to determine, depending on each corresponding patient's clinical information (22) of the patient, whether the corresponding predefined first sensor event has occurred and is relevant for diagnosis;
- labeling (130) the manufacturer information (20) based on a result of the analyzing;
- outputting data representative of the labeled manufacturer information (30) for diagnosis taking account of the predefined first sensor event occurrence and relevancy.

10. The method according to claim 9, wherein, for each patient:
- the at least one manufacturer information (20) comprises at least one alert information relating to a suspected abnormal activity of the patient's heart, each alert information being associated to a time stamp representing the time at which the alert information is generated;
- the patient's clinical information (22) comprises at least one intake of a drug and a corresponding drug intake time interval during which the patient takes said drug; and
- for each first type rule (21), the analysis comprises applying said first type rule (21) by comparing each time stamp to the drug intake time interval, so that whenever the time stamp is comprised in said drug intake time interval, the manufacturer information (20) corresponding to said time stamp is automatically labeled as labeled manufacturer information (30) not to be notified.

11. The method according to claim **10,** wherein the alert information includes an alert relating to an atrial fibrillation burden, and the drug is an anticoagulant drug.

12. The method according to any one of claims **9** to **11,** wherein, for each patient, the manufacturer information (20) further comprises at least one sensor information representative of a physiological and/or physical activity of the patient as a function of time, the analysis of the received manufacturer information (20) further comprising applying at least one second type rule (21) to said sensor information in order to determine whether a predefined second sensor event, associated to said second type rule (21), has occurred.

13. The method according to claim **12,** wherein the predefined second sensor event comprises at least one of the following medical conditions: first episode of atrial fibrillation, increasing paroxysmal atrial fibrillation, paroxysmal atrial fibrillation, persistent atrial fibrillation, and end of persistent atrial fibrillation.

14. The method according to claim **12,** wherein the at least one sensor information includes an atrial fibrillation burden as a function of time and/or a biventricular pacing percentage as a function of time, derived from the active implantable medical device.

15. The method according to any one of claims **9** to **14,** wherein, for each patient:
- the at least one manufacturer information (20) comprises:
• at least one alert information relating to a suspected abnormal activity of the patient's heart, each alert information being associated to a time stamp representing the time at which the alert information is generated, and
• for each alert information, at least one sensor information used to generate the alert information;
- the patient's clinical information (22) comprises at least one intake of a drug and a corresponding drug intake time interval during which the patient takes said drug;
- the analysis of the received at least one manufacturer information (20) further comprises applying at least one third type rule (21) to the received manufacturer information (20), each third type rule (21) being associated to a predefined third sensor event, and to at least one predefined threshold value, the applying each third type rule (21) to the received manufacturer information (20) including:
• comparing each time stamp to the drug intake time interval; and
• comparing a predetermined portion of each sensor information to the predefined threshold value,
so that, whenever the time stamp belongs to said drug intake time interval and the predetermined portion of the sensor information is lower or higher than the predefined threshold value, the manufacturer information (10) corresponding to said time stamp is automatically labeled as manufacturer information not to be notified.
